# EUROPEAN PATENT SPECIFICATION

(11) **EP 2 258 260 B1**
(45) Date of publication and mention of the grant of the patent: **15.02.2012**
(21) Application number: 10005524.3
(22) Date of filing: 27.05.2010
(51) Int. Cl.: A61B 5/022

(54) **Blood pressure cuff**
Blutdruckmanschette
Manchette pour pression artérielle

(30) Priority: 04.06.2009 JP 2009134713
(43) Date of publication of application: 08.12.2010
(73) Proprietor: Panasonic Electric Works Co., Ltd., Kadoma-shi Osaka (JP)
(72) Inventor: Kojima, Takeshi, Kadoma-shi, Osaka (JP); Fumuro, Shinichi, Kadoma-shi, Osaka (JP); Kanetsuna, Yoshitoshi, Kadoma-shi, Osaka (JP)
(74) Representative: Samson & Partner

(56) References cited:
- EP-A1- 1 593 340
- EP-A2- 1 632 174
- JP-A- 2009 066 199
- JP-A- 2009 077 814

## Description

### Field of the Invention

The present invention relates to a blood pressure cuff which is placed around an arm or wrist of a human body to restrict blood flow in a blood pressure measurement.

### Background of the Invention

In a blood pressure measurement, a blood pressure cuff is employed to restrict blood flow by wrapping the cuff around a human upper arm or wrist and inflating an air bag inside the cuff to occlude a corresponding artery. The air bag is connected to air supply tube for supplying air thereto. What is at issue here is the extending direction of the air supply tube from the air bag.

Specifically, when the blood pressure cuff is wrapped around the right upper arm of a user, if the air supply tube is extended toward the right wrist rather than the right shoulder, the air supply tube does not interfere with the placement of the cuff. On the other hand, when the blood pressure cuff is placed around the left upper arm of the user, if the air supply tube is extended toward the left wrist rather than the left shoulder, the air supply tube does not interfere with the placement of the cuff.

For that reason, there has been disclosed a blood pressure cuff according to the preamble of claim 1 including an air supply tube rotatably airtightly connected to a connector portion of an air bag, the extending direction of the air supply tube being changeable; and an indicator provided on an outer surface of the cuff to indicate around which upper arm and in which direction the cuff is required to be placed as the extending direction of the air supply tube is changed (see, e.g., Japanese Patent Application Publication No. 2009-077814).

In the blood pressure cuff, if the extending direction of the air supply tube is changed, the indicator indicates around which upper arm and in which direction the cuff is required to be placed, based on the changed direction.

In this way, it is possible to easily reliably place the blood pressure cuff around the left or right upper arm of a user without interference of the air supply tube to accurately measure the blood pressure.

However, since the blood pressure cuff has a shape that is not convenient enough to grab the cuff in the prior art, it takes a lot of time to place the cuff around the upper arm. Especially, when the blood pressure cuff is placed around the upper arm with a large load, for example, the blood pressure cuff is placed around a thick upper arm or the blood pressure cuff is placed around the upper arm while a sleeve is rolled up, this may require much time. Accordingly, there has been a demand for solving such a problem.

### Summary of the Invention

While the invention is defined in the claim 1, further aspects of the invention are set forth in the drawings and the following description.

In view of the above, the present invention provides a blood pressure cuff capable of being smoothly placed around an upper arm of a user and reducing the time for placing the cuff therearound.

In accordance with an embodiment of the present invention, there is provided a blood pressure cuff including an inflatable air bag provided inside the cuff to restrict blood flow; an air supply tube airtightly connected to a connector portion of the air bag, an extending direction of the air supply tube being changeable; an indicator provided on an outer surface of the cuff to indicate around which one of the left and right arm of a user and in which direction the cuff is required to be placed; and a handle provided to the indicator, the handle being used for pulling the cuff to place it around the left or right arm.

With such configuration, the indicator indicates around which upper arm and in which direction the cuff is required to be placed. Accordingly, it is possible to easily reliably place the blood pressure cuff around the arm to perform the accurate measurement. If the extending direction of the air supply tube is changed opposite to the indicated direction, the air supply tube does not interfere with the placement of the cuff.

Further, the blood pressure cuff can easily be pulled by a user gripping by the fingers the handle on the indicator in the side of the indicated arm. Accordingly, even when the blood pressure cuff is placed around the arm with a large load, the arm can smoothly be inserted into the blood pressure cuff and the time for placing the cuff can be reduced. In addition, the blood pressure cuff can be inexpensively realized by simply providing the handle in the indicator.

The blood pressure cuff further includes a mechanism for changing the extending direction of the air supply tube, the mechanism being cooperated with pulling of the handle to change the extending direction of the air supply tube opposite to the pulling direction of the handle.

With such configuration, the extending direction of the air supply tube is changed opposite to the pulling direction of the handle by the mechanism for changing the extending direction of the air supply tube, the mechanism being cooperated with the pulling of the handle. Accordingly, it is possible to automatically change the extending direction of the air supply tube.

### Brief Description of the Drawings

The objects and features of the present invention will become apparent from the following description of embodiments, given in conjunction with the accompanying drawings, in which:
Figs. 1A and 1B show a blood pressure cuff , wherein Fig. 1A is a perspective view thereof and Fig. 1B is a perspective view showing indicators each having a handle;
Fig. 2A is a cross sectional views of the blood pressure cuff and Fig. 2B is a partially enlarged cross sectional view showing a connector portion thereof and its vicinities;
Figs. 3A and 3B show a blood pressure cuff in accordance with the present invention, wherein Fig. 3A is a perspective view thereof and Fig. 3B is a perspective view of a indicator having a handle; and
Fig. 4A is a perspective view of an eccentric pin of a joint and Figs. 4B and 4C are side views showing a relationship between the an eccentric pin and a arc-shaped slot in accordance with the invention.

### Detailed Description of the Embodiments

Embodiments of the present invention will now be described with reference to the accompanying drawings which form a part hereof.

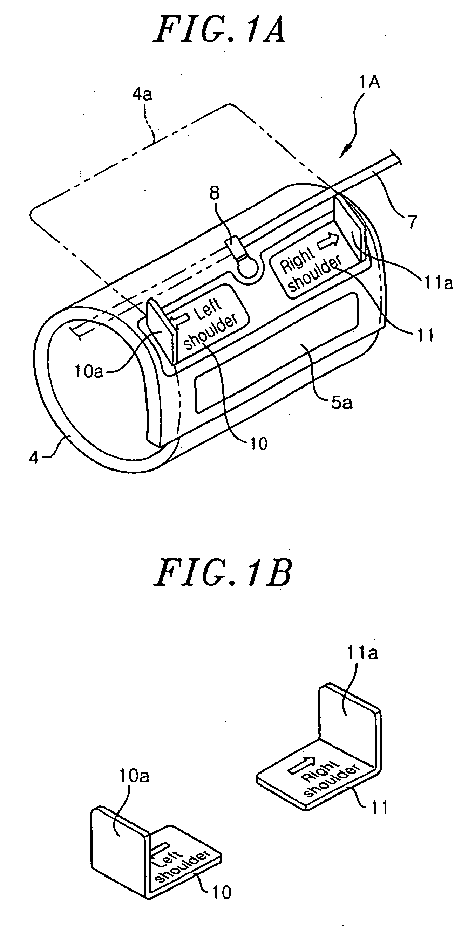

Figs. 1A to 2B show a blood pressure cuff 1A . The blood pressure cuff 1A is placed around an upper arm (or wrist) of a user by inserting it into the cuff 1A.

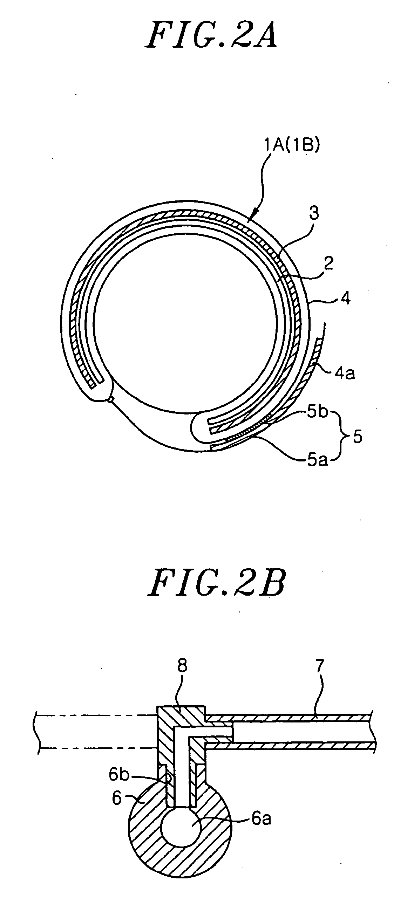

As shown in Fig. 2A, the blood pressure cuff 1A includes an air bag 2 for restricting blood flow, the air bag 2 being inflated by supplying air thereto by an air pump or like and deflated by exhausting the air therefrom through an exhaust valve or the like; and an elastic circular arc-shaped support plate (C-shaped clip) 3 arranged at an outer peripheral side of the air bag 2. The air bag 2 and the support plate 3 are accommodated in a cover strip 4.

When the blood pressure cuff 1A is placed around an upper arm of a user, the user inserts the hand into the blood pressure cuff 1A such that the blood pressure cuff 1A is placed around the upper arm while the support plate 3 maintaining the blood pressure cuff 1A in the substantially cylindrical shape is widened against its elastic force. Thereafter, the blood pressure cuff 1A is tightened by pulling a leading part 4a of the cover strip 4 and fixed by using a fastener 5 having a hook and a loop portion 5a and 5b.

As known in the art, by supplying air to inflate the air bag 2, the brachial artery is occluded to restrict the blood flow. Then, the blood pressure is measured as the air bag 2 is deflated by slowly exhausting the air therefrom.

As shown in Figs. 1A and 2B, the air bag 2 is provided with a connector 6 fixed to the support plate 3 and protruding through the cover strip 4 to the outside. Formed in the connector 6 is an air passageway 6a for allowing the air bag 2 to communicate with the outside.

To an opening 6b of the air passageway 6a formed at a side portion of the connector 6, a joint 8 that is coupled to a leading end of the air supply tube 7 is airtightly rotatably connected.

In Fig. 1A, the air supply tube 7 is extended to the right direction as the air supply tube 7 connected to the joint 8 is shown by a solid line and, when the joint 8 is rotated by 180°, the air supply tube 7 is extended to the left direction as shown by a dashed double-dotted line.

Indicators 10 and 11 are provided at opposite sides of the outer surface of the blood pressure cuff 1A in the width direction thereof, the indicators 10 and 11 being fixed to the support plate 3. In Fig. 1A, a left directional arrow and words "Left shoulder" are printed on the left indicator 10 and a right directional arrow and words "Right shoulder" are printed on the right indicator 11. The arrows and the words indicate around which one of the left and the right upper arm and in which direction the cuff 1A is required to be placed as will be described later.

The words printed on the indicators 10 and 11 are not limited to "Left shoulder" and "Right shoulder." Alternatively, letters "L" and "R," for example, may be printed.

Handles 10a and 11a are respectively provided to the indicator 10 and 11 as a single unit, the handles 10a and 11a extending upwardly so that the corresponding indicator and handle 10 and 10a or 11 and 11a have an approximately L-shaped cross section. A user may grip the handle 10a or 11a to pull up and place the blood pressure cuff 1A around the left or right arm.

When the blood pressure cuff 1A is placed around the right arm of a user, the joint 8 is rotated such that the air supply tube 7 is extended to the left direction (as shown by the dashed double-dotted line of Fig. 1A). Then, the user grips the handle 11a of the right indicator 11 on which the right directional arrow and the words "Right shoulder" are printed with the left fingers and pulls up and places the blood pressure cuff 1A around the right upper arm. Thereafter, the blood pressure cuff 1A is tightened by pulling the leading part 4a of the cover strip 4 and fixed around the right upper arm by using the fastener 5 provided in the cover strip 4.

On the other hand, when the blood pressure cuff 1A is placed around the left arm of the user, the joint 8 is rotated such that the air supply tube 7 is extended to the right direction (as shown by the solid line of Fig. 1A). Then, the user grips the handle 10a of the left indicator 10 on which the left directional arrow and the words "Left shoulder" are printed with the right fingers and pulls up and places the blood pressure cuff 1A around the left upper arm. Thereafter, the blood pressure cuff 1A is tightened by pulling the leading part 4a of the cover strip 4 and fixed around the left upper arm by using the fastener 5.

In the blood pressure cuff 1A , it is indicated, through the indicator 10 or 11, around which one of the left and the right arm and in which direction the cuff 1A is required to be placed. Accordingly, it is possible to easily reliably place the blood pressure cuff 1A around the left or right arm to perform the accurate measurement. Further, if the extending direction of the air supply tube 7 is changed opposite to the indicated direction, the air supply tube 7 does not interfere with the placement of the blood pressure cuff 1A.

In the meantime, it is not necessary to rotatably connect the joint 8 to the connector 6. Alternatively, the extending direction of the air supply tube 7 may be changed by bending the air supply tube 7 drawn to above the joint 8, for example.

Further, it is possible for a user to easily pull the blood pressure cuff 1A by gripping the handle 10a or 11a of the indicator 10 or 11 by the fingers. Accordingly, even when the blood pressure cuff 1A is placed around the arm with a large load, the arm can smoothly be inserted into the blood pressure cuff 1A and the time for placing the cuff 1A can be reduced. In addition, the cuff can be inexpensively realized by simply providing the handles 10a and 11a in the indicators 10 and 11, respectively.

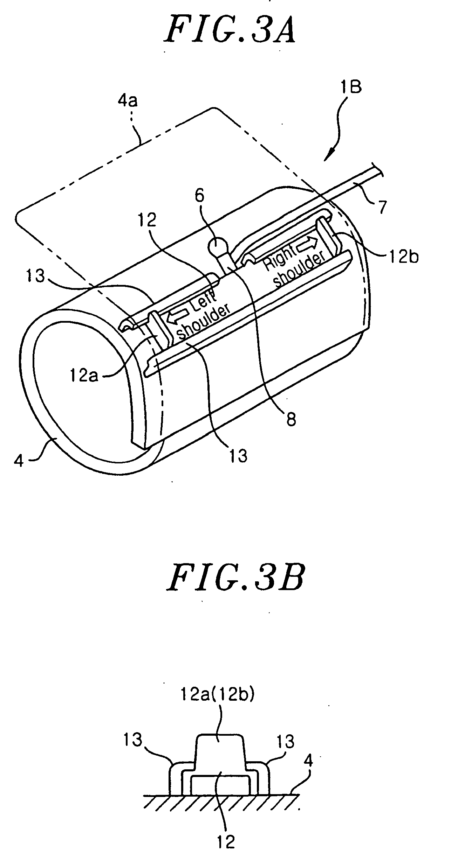

Figs. 3A to 4C show a blood pressure cuff 1B in accordance with the present invention. What is different from the blood pressure cuff 1A is the structure of an indicator 12.

Provided on an outer surface of the blood pressure cuff 1B is a rectangular-shaped indicator 12 extending in a width direction. The indicator 12 can slide in the width direction along a pair of rails 13 coupled to the support plate 3.

In Fig. 3A, a left directional arrow and words "Left shoulder" are printed at a left portion on the indicator 12 and a right directional arrow and words "Right shoulder" are printed at a right portion thereon.

Handles 12a and 12b are respectively provided at opposite sides of the indicator 12 as a single unit, the handles 12a and 12b extending upwardly.

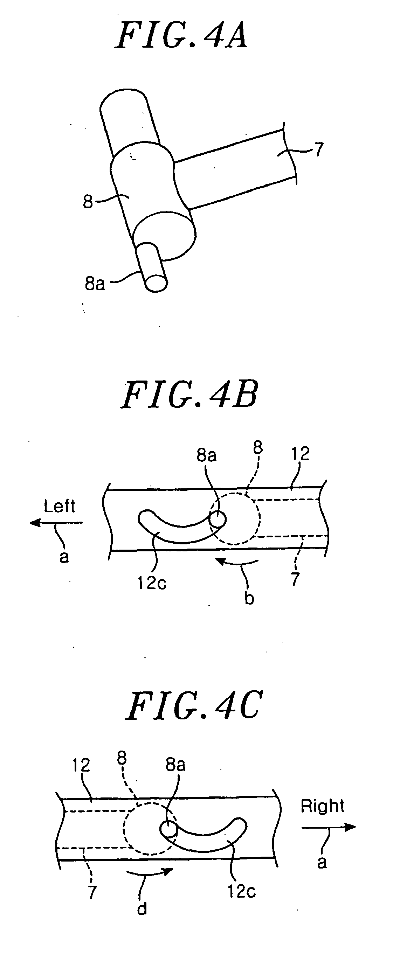

As shown in Fig. 4A, an eccentric pin 8a is provided on an outer bottom surface of the joint 8 of the air supply tube 7, the joint 8 being rotatably connected to the connector 6.

An arc-shaped groove 12c is formed on a longitude side surface of the indicator 12, which faces the outer bottom surface of the joint 8, as shown in Fig. 4B. The eccentric pin 8a of the joint 8 is engaged with the arc-shaped slot 12c. A mechanism for changing the extending direction of the air supply tube 7 is constituted by the eccentric pin 8a and the arc-shaped slot 12c.

When the blood pressure cuff 1B is placed around the left arm of a user, the users grips by right fingers the left handle 12a upwardly extending at the left side of the indicator 12 on which the left arrow and the words "Left shoulder" are printed and pulls the blood pressure cuff 1B in the direction of the left upper arm, so that the indicator 12 slides in the left direction (i.e., the direction of an arrow "a") as shown in Fig. 4B.

At this time, the joint 8 is rotated in the right direction (i.e., the direction of an arrow "b" shown in Fig. 4B) through the eccentric pin 8a engaged with the arc-shaped slot 12c. Accordingly, the extending direction of the air supply tube 7 is automatically changed to the right direction.

On the other hand, when the blood pressure 1B is placed around the right arm of the user, the user grips by left fingers the right handle 12b upwardly extending at the right side of the indicator 12 on which the right arrow and the words "Right shoulder" are printed and pulls up the blood pressure cuff 1B in the direction of the right upper arm, so that the indicator 12 slides in the right direction (i.e., the direction of an arrow "c") as shown in Fig. 4C.

At this time, the joint 8 is rotated in the left direction (i.e., the direction of an arrow "d" shown in Fig. 4C) through the eccentric pin 8a engaged with the arc-shaped slot 12c. Accordingly, the extending direction of the air supply tube 7 is automatically changed to the left direction.

In the blood pressure cuff 1B , the extending direction of the air supply tube 7 is changed opposite to the pulling direction of the handle 12a or 12b by the mechanism for changing the extending direction of the air supply tube 7, which includes the eccentric pin 8a and the arc-shaped slot 12c, the mechanism being cooperated with the pulling of the handle 12a or 12b. Accordingly, it is possible to automatically change the extending direction of the air supply tube 7.

While the invention has been shown and described with respect to an embodiment, it will be understood by those skilled in the art that various changes and modifications may be made without departing from the scope of the invention as defined in the following claim.

## Claims

1. A blood pressure cuff comprising:
an inflatable air bag (2) provided inside the cuff to restrict blood flow;
an air supply tube (7) airtightly connected to a connector portion (6) of the air bag, an extending direction of the air supply tube (7) being changeable;
an indicator (12) provided on an outer surface of the cuff to indicate around which one of the left and right arm of a user and in which direction the cuff is required to be placed; and
a handle (12a or 12b) provided to the indicator (12), the handle (12a or 12b) being used for pulling the cuff to place it around the left or right arm,
**characterized in that** the blood pressure cuff further comprises a mechanism (8) for changing the extending direction of the air supply tube (7), wherein the mechanism (8) is cooperated with pulling of the handle to change the extending direction of the air supply tube (7) opposite to the pulling direction of the handle (12a or 12b).

## Patentansprüche

1. Blutdruckmanschette, enthaltend:
einen aufblasbaren Luftsack (2), der innerhalb der Manschette vorgesehen ist, um den Blutstrom zu drosseln;
einen Luftzuführungsschlauch (7), der mit einem Anschlußbereich (6) des Luftsacks luftdicht verbunden ist, wobei eine Richtung, in der sich der Luftzuführungsschlauch (7) erstreckt, veränderbar ist;
eine Anzeigevorrichtung (12), die an einer äußeren Oberfläche der Manschette vorgesehen ist, um anzuzeigen, um welchen von dem linken und dem rechten Arm eines Benutzers und in welcher Richtung die Manschette angelegt werden soll; und
eine Handhabe (12a oder 12b), die an der Anzeigevorrichtung (12) vorgesehen ist, wobei die Handhabe (12a oder 12b) verwendet wird, um die Manschette an ihren Platz um den linken oder rechten Arm zu ziehen,
**dadurch gekennzeichnet, dass** die Blutdruckmanschette weiterhin einen Mechanismus (8) umfasst zum Ändern der Erstreckungsrichtung des Luftzuführungsschlauchs (7), wobei der Mechanismus (8) mit dem Ziehen der Handhabe zusammenwirkt, um die Erstreckungsrichtung des Luftzuführungsschlauchs (7) entgegen die Zugrichtung der Handhabe (12a oder 12b) zu ändern.

## Revendications

1. Brassard destiné à mesurer la pression artérielle comprenant :
✔ une poche d'air gonflable (2) disposée à l'intérieur du brassard destinée à limiter le flux sanguin ;
✔ un tube d'alimentation en air (7) relié de manière étanche à une partie de raccord (6) de la poche d'air, la direction de déploie-ment du tube d'alimentation en air (7) pouvant être modifiée ;
✔ un indicateur (12) disposé sur une surface extérieure du brassard destiné à indiquer autour de quel bras, gauche ou droit, d'un patient et dans quelle direction, il convient de placer le brassard ; et
✔ une poignée (12a ou 12b) disposée sur l'indicateur (12), la poignée (12a ou 12b) étant utilisée de façon à tirer le brassard afin de le placer autour du bras gauche ou du bras droit ;
**caractérisé en ce que** le brassard destiné à mesurer la pression artérielle comprend en outre un mécanisme (8) destiné à modifier la direction de déploiement du tube d'alimentation en air (7), le mécanisme (8) coopérant avec la traction de la poignée de façon à modifier la direction de déploiement du tube d'alimentation en air (7) à l'opposé de la direction de traction de la poignée (12a ou 12b).
